# EUROPEAN PATENT SPECIFICATION

(11) **EP 0 871 441 B1**
(45) Date of publication and mention of the grant of the patent: **13.08.2003**
(21) Application number: 97910042.7
(22) Date of filing: 03.10.1997
(51) Int. Cl.: A61K 31/335, A61K 31/195, A61P 35/00

(54) **DFMO AND TAXOL FOR THE TREATMENT OR PREVENTION OF BREAST CANCER**
DFMO und Taxol zur Behandlung bzw. Vorbeugung von Brustkrebs
DFMO ET TAXOL POUR LE TRAITEMENT OU LA PREVENTION DU CANCER DU SEIN

(30) Priority: 04.10.1996 US 27837 P
(43) Date of publication of application: 21.10.1998
(73) Proprietor: Ilex Oncology, Inc., San Antonio, TX 78230-1064 (US)
(72) Inventor: WEIS, Alexander, San Antonio, TX 78257 (US); LOVE, Richard, San Antonio, TX 78257 (US); MANGOLD, Gina, Castroville, TX 78009 (US)
(74) Representative: Klunker . Schmitt-Nilson . Hirsch
(86) International application number: US9718252
(87) International publication number: WO98014188

(56) References cited:
- EP-A- 0 580 507
- Database Cancerlit Abstract accession no. 9763736 & Proc.Annu.Meet.Am.Assoc.Cancer Res., vol. 38, 1997, Abstract no. A636 XP002053563
- Database Medline Abstract accession no. 90014698 & Nippon Geka Gakkai Zasshi vol. 90, no. 5, 1989, pages 650-60 XP002053564
- MASAKUNI NOGUCHI ET AL.: "Breast cancer chemoprevention: Clinical Trials and research" ONCOLOGY, vol. 53, no. 3, 1996, pages 175-181, XP002053560
- THRESIA THOMAS ET AL.: "Additive growth-inhibitory effects of DL-alpha-difluoromethylornithine and antiestrogens on MCF-7 breast cancer cell line" BIOCHEM.BIOPHYS.RES.COMMUN., vol. 148, no. 3, 1987, pages 1338-1345, XP002053561
- PRASAD S. SUNKARA ET AL.: "Inhibition of polyamine biosynthesis by alpha-difluoromethyl ornithine potentiates the cytotoxic effects of arabinosyl cytosine in HELA cells" BIOCHEM.BIOPHYS.RES.COMMUN., vol. 95, no. 1, 1980, pages 423-430, XP002053562

## Description

### FIELD OF THE INVENTION

The present invention provides a pharmaceutical formulation a kit of parts comprising said formulation for use as a medicament, and the use of said formulation for the manufacture of a medicament for the treatment of cancer. More specifically, the present invention provides a composition comprising alpha difluoromethylornithine for use in treating, preventing, reducing the risk of and/or controlling the growth of estrogen independent breast cancers and solid tumors.

### BACKGROUND OF THE INVENTION AND DESCRIPTION OF THE PRIOR ART

ODC (omithine decarboxylase) catalyzes the first step in the biosynthesis of putrescine (a diamine), spermidine and spermine, the three major polyamines of mammalian cells. *In vitro* studies show that polyamines participate in nearly all aspects of DNA, RNA, and protein synthesis. Polyamine accumulation is required to maintain maximum rates of cell proliferation. Blockage of polyamine accumulation by administration of DFMO and other inhibitors during accelerated cell growth results in a significant reduction of growth in a variety of cell systems. Since the only pathway to polyamine synthesis is via ornithine, synthesis depends on the activity of ODC. ODC is present in very small amounts in resting cells but can be increased many-fold within a few hours of exposure to hormones, drugs, and growth factors.

DFMO has been shown to inhibit cellular replication in vitro in several malignant animal tumor cell lines, including L1210 and LS178Y leukemia, rat hepatoma, mouse mammary sarcoma (EMT6) and hamster pancreatic adenocarcinoma (H2T) (Mamont et al., 1978; Prakash et al., 1980; Pera et al., 1986; Marx et al., 1987).

DFMO, either alone or in combination with other agents, has reportedly proven effective in treating and/or preventing mammary carcinomas in animal models as follows. The growth of six human tumors (three mammary carcinomas, a malignant melanoma, a bladder carcinoma, and an endocervical carcinoma) was significantly decreased after DFMO treatment compared to growth in control mice. (Luk et al., 1983). In xenografts of human breast and colon carcinoma cells inoculated into nude mice, a synergistic antitumor effect was observed when DFMO was combined with mitomycin D (Takami et al., 1989).

DFMO, either alone or in combination with other agents, has also reportedly demonstrated some activity in animal models or cell lines in controlling the growth of both estrogen dependent (responsive, ER+) and estrogen independent (unresponsive, ER-) tumor cell lines.

The growth inhibition of ZR-75-1 cells, a line of ER+ human breast cancer cells in culture, caused by tamoxifen, an anti-estrogen, alone or in combination with DFMO has been reported. (Hoggard et al., 1986)

All-trans-retinoic acid (RA) inhibited estradiol (ES, Steraloids) stimulated proliferation of the estrogen-dependent breast carcinoma cell lines MCF-7 and ZR-75. (Fontana et al., 1990) This effect was temporally associated with a marked stimulation of the synthesis and secretion of a 75,000 molecular weight (MW) protein. Stimulation of this 75,000 MW protein in MCF-7 cells occurred at low RA levels, in both the presence and absence of ES but not in the presence of tamoxifen (TMX) or DFMO.

Retinoic acid (RA) reportedly inhibited proliferation of numerous breast carcinoma cells and prevented estrogen stimulation of growth of several estrogen receptor (ER)-positive cell lines. (Fontana et al., 1990) RA inhibition of ER+ human breast carcinoma cell proliferation was associated with marked inhibition of the synthesis of a M(r) 39,000 protein in the ER-positive human breast carcinoma cell lines investigated. Tamoxifen, which inhibits ER-positive breast carcinoma proliferation, moderately inhibited M(r) 39,000 synthesis, while a concentration of difluoromethylornithine which inhibits cellular proliferation by greater than 50% did not affect M(r) 39,000 protein synthesis.

The antiproliferative effects of 4-hydroxy-tamoxifen and DFMO have been tested. (Cohen et al., 1990) The antiestrogen 4-hydroxy-tamoxifen and DFMO inhibited MCF-7 growth, and this effect was additive.

Further, estrogenic and antiestrogenic responses were investigated in human breast MCF-7 cancer cells. (Kendra, 1988) Use of DFMO, indicated that the integrity of the polyamine biosynthetic pathway was essential for estrogenic regulation of cell proliferation. Yet, the antiproliferative effects of antiestrogens were not due to depletion of intracellular polyamine levels. However, DFMO reportedly had no effect on estrogenic regulation of progesterone receptor levels. The antiestrogen (AE), tamoxifen, acted as a partial estrogen agonist and antagonist in terms of both cell proliferation and cell cycle kinetics.

The growth-inhibitory effects of DFMO and antiestrogens (tamoxifen, 4-hydroxytamoxifen, trioxifene, keoxifene, and LY 117018) as single agents and in combinations on the proliferation of a breast cancer cell line, MCF-7 were further studied (Thomas, 1987). At 0.1 mM DFMO, the proliferation of MCF-7 cells was inhibited to 75 % of the controls. Treatment of the cells with 0.1µM 4-hydroxytamoxifen reduced cell growth to 72%. Combination of 0.1 mM difluoromethylornithine and 0.1µM 4-hydroxytamoxifen reduced cell growth to 38%, indicating additive growth-inhibitory effects. Similar additive effects were observed with all 5 antiestrogens in combination with difluoromethylomithine.

Treatment of MCF-7 cells with DFMO reportedly prevented estradiol-induced cell proliferation in a dose-dependent fashion. (Kendra, 1987) DFMO inhibition of estradiol-induced cell proliferation was completely recoverable by the addition of exogenous putrescine, whereas putrescine alone did not stimulate proliferation of control cells. ODC activity was 4-fold greater in estrogen-treated cells, and DFMO (5 mM) fully inhibited ODC activity. DFMO was able to suppress only slightly further the proliferation of tamoxifen-treated cells and putrescine was able to recover this DFMO inhibition. In contrast to the suppressive effect of DFMO on cell proliferation, DFMO had no effect on the ability of estrogen to stimulate increased (4-fold elevated) levels of progesterone receptor. Hence, while ODC activity appears important for estrogen-induced cell proliferation, inhibition of the activity of this enzyme has no effect on the ability of estradiol to increase cellular progesterone receptor content.

An efficient route and dose regime for the long-term administration of tamoxifen in the study of mammary tumorigenesis in the rat were studied. (Thompson, 1986) The objective of this work was to determine whether treatment with DFMO would reduce the occurrence of mammary cancers in tamoxifen-treated or ovariectomized rats. A total of 265 female Sprague-Dawley rats were injected with 50 mg 1-methyl- 1 -nitrosourea (MNU)/kg at 50 days of age. In a first experiment, beginning 7 days after the injection of the carcinogen, animals were assigned to 1 of 6 groups which received either 0, 1 or 5 mg tamoxifen citrate/kg AIN-76A purified diet in addition to either no DFMO or a 0.125% solution of DFMO as the drinking water. The experiment was terminated 180 days following carcinogen treatment. Treatment with tamoxifen resulted in a dose-dependent reduction in cancer incidence, the number of cancers induced, and significantly prolonged the median cancer-free time. This effect was also accompanied by a decrease in the rate of body weight gain. Treatment with DFMO delayed latency and reduced tumor number. DFMO in addition to tamoxifen (1 mg/kg diet) further prolonged latency. In a second experiment, each animal was assigned to 1 or 4 treatment groups when its first palpable mammary tumor was detected. At that time, each was either ovariectomized or sham-operated. In addition, the rats were either provided no DFMO or a 0.5% solution of DFMO as the drinking water. The study was terminated 35 weeks following carcinogen injection. Ovariectomy significantly inhibited the occurrence of additional mammary tumors. Ovariectomy plus DFMO was more effective than ovariectomy alone in reducing tumor number. According to the authors, these observations indicate that suppression of polyamine biosynthesis vh the systemic administration of DFMO inhibits the development of ovarian hormone insensitive mammary tumors.

ODC inhibition using DFMO reportedly inhibited the proliferation of estrogen-responsive cell-lines MCF-7 and T-47D by 80% and the estrogen-unresponsive cell line BT-20 by 50%. (Thomas, 1985) Suppression of MCF-7 cell growth by tamoxifen reportedly could be rescued by polyamines. Hydroxytamoxifen and DFMO had a synergistic growth inhibitory effect on MCF-7 and T-47D cells.

Taxol alone and in combination with other agents has been evaluated for the treatment of cells from metastatic breast cancer, breast tumor, refractory breast cancer, estrogen responsive and unresponsive breast cancer, advanced breast cancer and high risk breast cancer. Such other agents have included gamma-CSF, epirubicin, edatrexate, yttrium-90 DOTA peptide chimeric L6, vinorelbine, ifosfamide, novantrone, thiotepa, cisplatin, etoposide, cyclophosphamide, epirubicin, platinum, adriamycin and others. Results from these studies have varied widely.

Thus, the prior art contemplates treating and/or preventing breast cancer by the administration of DFMO either alone or in combination with other agents and the administration of taxol either alone or in combination with other agents. However, there has been no teaching or suggestion of treating breast cancer with the combination of taxol and DFMO. There is also little information available on the treatment of ER- breast cancer with DFMO alone and no prior teaching or suggestion of treatment of ER- breast cancer with DFMO in combination with taxol. There is also no teaching in the prior art of a composition containing a single enantiomer or a defined ratio of enantiomers of DFMO for treating, preventing, controlling the growth of and/or reducing the risk of developing breast cancer and solid tumor. Thus, the need remains for an effective therapy for treating breast cancer and solid tumor.

### SUMMARY OF THE INVENTION

In one embodiment, the present invention provides a pharmaceutical composition for use as a medicament, in particular for use in treating, preventing, controlling the growth of and/or reducing the risk of developing breast cancer and solid tumor comprising therapeutic amounts of taxol, or one of its derivatives or metabolites, and (+)-DFMO, (-)-DFMO or a mixture of (+)-DFMO: (-)-DFMO, or pharmaceutically acceptable salts thereof, and a pharmaceutically acceptable carrier.

It is also contemplated and within the scope of the invention that the pharmaceutical composition can further comprise one or more other therapeutic compounds or cytotoxic agents for treating, preventing, reducing the risk of or controlling the growth of breast cancer or solid tumor.

Another embodiment of the present invention provides the use of a therapeutically effective amount of taxol, or one of its derivatives or metabolites, and (+)-DFMO, (-)-DFMO or a mixture of (+)-DFMO and (-)-DFMO, or pharmaceutically acceptable salts thereof and a pharmaceutically acceptable carrier, for the manufacture of a medicament treating, preventing, controlling the growth of and/or reducing the risk of breast cancer and tumor.

Yet another aspect of the invention provides a kit of parts. In one embodiment, the kit comprises a therapeutically effective amount of taxol or a derivative, prodrug or metabolite thereof, and (+)-DFMO, (-)DFMO, or a mixture of (+)-DFMO and (-)-DFMO, or pharmaceutically acceptable salts thereof, for use in sequential or concurrent administration as a medicament. The kit in other embodiments may be further defined as one for use in treating, preventing, controlling the growth of and/or reducing the risk of breast cancer or tumor. In yet another aspect, the kit may be defined as one comprising a component of parts suitable for use in treating a patient having or being at risk of developing breast cancer. The kit in yet another aspect may be further defined as one comprising a component of parts suitable for use in preventing breast cancer or tumor growth. The kit may also be described in other aspects as comprising a component of parts suitable for use in controlling and/or inhibiting the growth of breast cancer or tumor. In yet further embodiments, the kit is defined as providing a preparation for use in reducing the risk of estrogen independent breast cancer or tumor growth.

The various kits of the invention may also include a pharmaceutically acceptable carrier solution. By way of example, such a pharmaceutically acceptable carrier solution comprises saline.

Other features, advantages and embodiments of the invention will be apparent to those skilled in the art from the following description, accompanying data and appended claims.

### BRIEF DESCRIPTION OF THE DRAWINGS

The following drawings form part of the present specification and are included to further demonstrate certain aspects of the invention. The invention may be better understood by reference to one or more of these drawings in combination with the detailed description of specific embodiments presented herein.

Fig. 1. Response curve for the treatment of MCF-7 human breast tumor with DFMO and taxol alone and in combination (-●- = control; -▲- = DFMO (0.5%); -◆- = taxol 16; 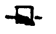 = DFMO + Taxol; -■- = E2 Control).

Fig. 2. Response curve for the treatment of MDA-MB-231 human breast tumor with DFMO and taxol alone and in combination (-●- = control; -■- = 3% DFMO; 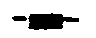 = Taxol (20 mg/kg; -▲- = combination).

### DETAILED DESCRIPTION OF THE PREFERRED EMBODIMENTS

### Treating, Preventing, Controlling the Growth of and Reducing the Risk of ER-Breast Cancer

In each of the above indications, racemic DFMO has been reported to yield non-conclusive effect on various neoplastic disorders. Administration of a single enantiomer or a defined non-racemic ratio or mixtures of enantiomers of DFMO to treat, prevent or slow the growth of cancers/tumors will provide an enhanced pharmacological activity in a patient as compared to administration of racemic DFMO. Evidence for such an enhanced pharmacological actively can be found by employing methods and preparations as described herein.

By "enhanced pharmacological activity" is meant improved bioavailability, reduced toxicity or side effects, enhanced therapeutic or clinical benefit and/or enhanced pharmacological profile.

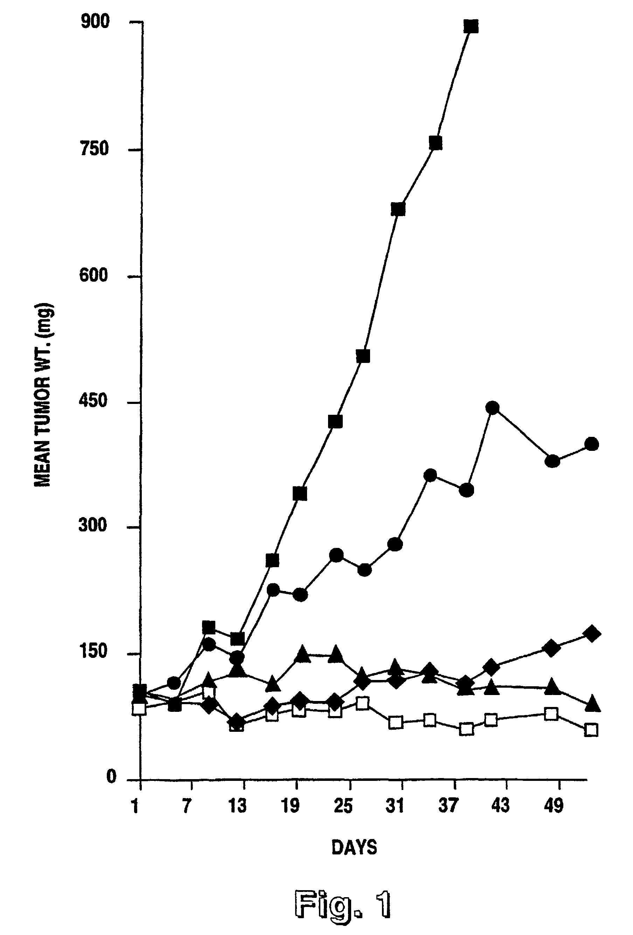

Referring now to Figure 1 and Table 2 (Example 6), DFMO and taxol, alone and in combination, were evaluated for the treatment of MCF-7 breast cancer, MCF-7 is an ER+ cell line. Control animals, as indicated by the filled circles, were given no drug. A second group of animals, as indicated by the filled squares, was administered estradiol (E2) as a positive control in this model. A third group of animals, as indicated by the filled triangles, was administered DFMO alone (0.5 % wt in drinking water, p.o. ad libitum) which provided a 44% tumor growth inhibition (TGI) with respect to (wrt) control. A fourth group of animals, as indicated by the filled diamonds, was administered taxol alone (16 mg/kg, i.p., qdx5) which provided a 28% TGI wrt control. The last group of animals, as indicated by the half-filled squares, was administered DFMO and taxol in combination (0.5 % wt in drinking water, p.o., ad libitum; 16 mg/kg, i.p., qdx5, respectively) which provided an 89 % TGI wrt control.

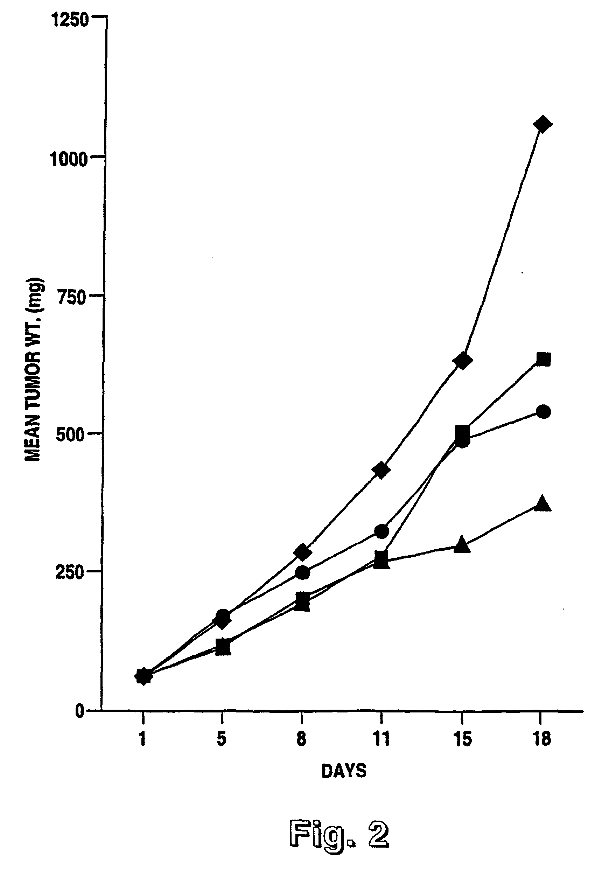

Referring now to Figure 2 and Table 1, DFMO and-taxol, alone and in combination, were evaluated according to the method of Example 4 for the treatment of MDA-MB-23 1 breast cancer. This is an ER- cell line. Control animals were given no drug. A second group of animals was administered DFMO alone (0.5 % wt in drinking water, p.o. ad libitum) and showed a 19 % tumor growth inhibition (TGI) with respect to (wrt) control. A third group of animals was administered DFMO alone (3.0% wt. in drinking water, p.o. ad libitum) and showed a *52%* TGI wrt control. A fourth group of animals was administered taxol alone (20 mg/kg, i.p., qdx5) and showed 84 % TGI wrt control. A fifth group of animals was administered DFMO and taxol in combination (0.5% wt. in drinking water, p.o., ad libitum; 20 mg/kg, i.p. qd x 5, respectively) and showed a 90% TGI. The last group of animals was administered DFMO and taxol in combination (3.0 % wt in drinking water, p.o., ad libitum; 20 mg/kg, i.p., qdx5, respectively). All animals died during the study. DFMO alone and also in combination with taxol will be useful not only in treating breast cancer but also in delaying its growth or onset.

The present data demonstrates the utility of DFMO in treating, preventing the occurrence of, controlling the growth of and reducing the risk of developing ER-breast cancer and tumor.

### Combination therapy

The anti-tumor effect of DFMO in combination with various cytotoxic agents has reportedly been demonstrated as follows: (a) in combination with vindesine or adriamycin in L1210 leukemia in mice, in Morris 7288C hepatoma in Buffalo rats, and in EMT6 tumor in mice, (b) in combination with cytosine arabinoside in L1210 leukemia in mice, (c) in combination with methotrexate in L1210 leukemia in mice, (d) in combination with cyclophosphamide in EMT6 tumor in mice and in DMBA-induced tumor in mice, (e) in combination with BCNU in mouse glioma 26 brain tumor, and (f) in combination with MGBG in L1210 leukemia in mice, in Morris 7288C hepatoma.

As used herein, the term "tumor" means both benign and malignant tumors or neoplasms, and includes melanomas, lymphomas, leukemias, and sarcomas. Illustrative examples of tumor tissues are cutaneous tumors, such as malignant melanomas and mycosis fungoides; hematologic tumors such as leukemias, for example, acute lymphoblastic, acute myelocytic or chronic myelocytic leukemia; lymphomas, such as Hodgkin's disease or malignant lymphoma; gynecologic tumors, such as ovarian and uterine tumors; urologic tumors, such as those of the prostate, bladder or testis; soft tissue sarcomas, osseus or non-osseus sarcomas, breast tumors; tumors of the pituitary, thyroid and adrenal cortex; gastrointestinal tumors, such as those of the esophagus, stomach, intestine and colon; pancreatic and hepatic tumors; laryngeae papillometastases and lung tumors.

The term "controlling the growth", as used herein, means slowing, inhibiting interrupting, arresting, or stopping the growth and mctastases of a rapidly proliferating tumor or cancer in a warm blooded animal. It should be understood that treatment (controlling the growth of a tumor tissue) in a warm blooded animal with DFMO enantiomers, either with or without the added effects of an additional cytotoxic agent or therapeutic drug, generally provides a clinical benefit such as improved survivability or quality of life although the tumor tissue may not necessarily be completely destroyed or totally eliminated. Experimentally, however, some tumor tissues have been completely eliminated.

As used herein, the term "reducing the risk of" means reducing the incidence or rate of occurrence or reoccurrence, of a given disorder in a patient receiving DFMO therapy. As used herein, the term "preventing" means eliminating the incidence of a given disorder in a patient receiving DFMO therapy.

As used herein the term "patient" includes warm blooded animals such as mammals, for example, dogs, rats, mice, cats, guinea pigs, horses, bovine cows, sheep, and humans.

DFMO can be administered in conjunction with surgical excision of the tumor or with radiation therapy, immunotherapy, local heat therapy, or any combination thereof. DFMO can also be administered to a patient in combination with a chemical cytotoxic agent known in the art to be useful for tumor therapy. When such combination therapy is employed for the treatment of a tumor, the cytotoxic agent may be administered at a dosage known in the art to be effective for treating the tumor.

It is likely that specific combinations of DFMO and another cytotoxic agent will prove particularly effective at treating, preventing, controlling the growth of and/or reducing the risk of estrogen independent breast cancer or tumor.

When, in the treatment of an ER-human breast cancer, DFMO is administered in combination with a cytotoxic agent such as taxol, the therapeutic effect of the cytotoxic agent can be potentiated. The remission produced by the cytotoxic agent can be enhanced and regrowth of the tumor or cancer tissue can be slowed or prevented. Use of such combination therapy allows smaller doses or fewer individual doses of the cytotoxic agent to be employed. The detrimental and/or debilitating side effects of the cytotoxic agent may be minimized while the anti-tumor effects are enhanced.

The term "combination therapy" contemplates the administration of DFMO prior to the beginning of therapy with a cytotoxic agent, concomitantly with such therapy, or during the period of time following cessation of such therapy. In one embodiment, the patient may be treated with DFMO for about 1 to 14 days, preferably 4 to 14 days, prior to the beginning of therapy with a cytotoxic agent, and thereafter, on a daily basis during the course of such therapy. Daily treatment with DFMO can be continued for a period of, for example, 1 to 365 days after the last dose of the cytotoxic agent is administered.

When such combination therapy results in remission of the tumor or cancer, and all tumor or cancer cells are not destroyed, regrowth of the tumor or cancer may be prevented or slowed indefinitely by continued treatment with DFMO.

As used herein, the term "therapeutic compound" is a compound having the desired beneficial pharmacologic and therapeutic effects in mammals. Advantageously, the therapeutic compound is a cytotoxic agent and is also indicated for the treatment or prophylaxis of cancer and/or tumors.

The therapeutic compounds contemplated within the scope of the invention may be in their free acid, free base, or pharmaceutically acceptable salt forms. They may be derivatives or prodrugs of a given compound.

Loading of the therapeutic compounds into a pharmaceutical formulation may be accomplished following well known techniques such as those described Remington's Pharmaceutical Sciences, 17th ed., Mack Publishing Company, Easton, PA, 1985, the disclosure of which is hereby incorporated by reference.

Therapeutic compound loading into the formulation may need to be varied according to the pharmacological activity of the compound, the indication being treated, the targeted dosing regimen, the projected method of administration, the integrity or stability of the final formulation or other such reasons.

Illustrative examples of cytotoxic agents or therapeutic compounds which can be administered in combination with DFMO include, by way of example and without limitation:
Alkylating agents;
Alkyl Sulfonates such as Busulfan, lmprosulfan, Piposulfan;
Aziridines such as Benzodopa, Carboquone, Meturedopa, Uredopa;
Ethylenimines and Methylmelamines such -as Altretamine, Triethylenemelamine, Triethylenephosphoramide, Triethylenethiophosphoramide, Trimethylolomelamine;
Nitrogen Mustards such as Chlorambucil, Chlornaphazine, Cholophosphamide, Estramustine, Ifosfamide, Mechlorethamine, Mechlorethamine Oxide Hydrochloride, Melphalan, Novembichin, Phenesterine, Prednimustine, Trofosfamide, Uracil Mustard;
Nitrosoureas such as Carmustine, Chlorozotocin, Fotemustine, Lomustine, Nimustine, Ranimustine;
Antibiotics such as Aclacinomycins, Actinomycin F₁, Authramycin, Azaserine, Bleomycins, Cactinomycin, Carubicin, Carzinophilin, Chromomycins, Dactinomycin, Daunorubicin, 6-Diazo-5-oxo-L-norleucine, Doxorubicin, Epirubicin, Mitomycins, Mycophenolic Acid, Nogalamycin, Olivomycins, Peplomycin, potfiromycin, Puromycin, Streptonigrin, Streptozocin, Tubercidin, Ubenimex, Zinostatin, Zorubicin;
Antimetabolites;
Folic Acid Analogs such as Denopterin, Methotrexate, Pteropterin, Trimetrexate;
Purine Analogs such as Fludarabine, 6-Mercaptopurine, Thiamiprine, Thioguanine;
Pyrimidine Analogs such as Ancitabine, Azacitidine, 6-Azauridine, Carmofur, Cytarabine, Dideoxyuridines, Doxifluridine, Enocitabine, Floxuridine, Fluorouracil, Tegafur;
Others such as Aceglatone, Aldophosphamide Glycoside, Aminolevulinic Acid, Amsacrine, Bestrabucil, Bisantrene, Carboplatin, Cisplatin, Defofamide, Demecolcine, Diaziquone, Elfornithine, Elliptinium Acetate, Etoglucid, Etoposide, Gallium Nitrate, Hydroxyurea, Interferon-α, Interferon-β, Interferon-γ, Interleukine-2, Lentinan, Lonidamine, Mitoguazone, Mitoxantrone, Mopidamol, Nitracrine, Pentostatin, Phenamet, Pirarubicin, Podophyllinic Acid, 2-Ethylhydrazide, Procarbazine, PSK®, Razoxane, Sizofiran, Spirogermanium, Taxol, Tamoxifen, Teniposide, Tenuazonic Acid, Triaziquone, 2,2'2"-Trichlorotriethylamine, Urethan, Vinblastine, Vincristine, Vindesine, Dacarbazine, Mannomustine, Mitobronitol, Mitolactol, and Pipobroman;
Androgens such as Calusterone, Dromostanolone Propionate, Epitiostanol, Mepitiostane, Testolactone;
Antiadrenals such as Aminoglutethimide, Mitotane, Trilostane;
Antiandrogens such as Flutamide, Nilutamide;
Antiestrogens such as Aromatase Inhibiting 4(5)-Imidazoles; and
Folic Acid Replenisher such as Frolinic Acid.

The therapeutic compound(s) contained within the formulation may be formulated as their pharmaceutically acceptable salts. As used herein, "pharmaceutically acceptable salts" refer to derivatives of the disclosed compounds wherein the parent pharmacologically active compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfonic, sulfamic, phosphoric, nitric and the like; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent pharmacologically active compound which contains a basic or acidic moiety by conventional chemical methods. Generally, such salts can be prepared by reacting the free acid or base forms of these compounds with a predetermined amount of the appropriate base or acid in water or in an organic solvent, or in a mixture of the two. Generally, nonaqueous media are preferred. Lists of suitable salts are found in Remington's Pharmaceutical Sciences, 17th ed., Mack Publishing Company, Easton, PA, 1985, p. 1418, the disclosure of which is hereby incorporated by reference.

The phrase ''pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

It should be understood that methods for the treatment of estrogen independent breast cancer will employ a variety of formulations, dosage forms and dosing regimens, i.e., each formulation will have associated with it an optimal dosage form and dosing regimen.

### Dosage Form

The pharmaceutical composition of the present invention can be administered by a variety of routes such as, by way of example and without limitation: intraperitoneal, intra-articular, intra-arterial, intracardiac, intracavity, intradermal, intrathecal, intrathoracic, percutaneous, intravascular, intravenous, intracoronary, intramuscular or subcutaneous injection; or oral, buccal, rectal or sublingual administration. Such methods of administration and others contemplated within the scope of the present invention are known to the skilled artisan. When used to prevent or reduce the risk of estrogen independent breast cancer, DFMO will generally be administered chronically and at lower doses than those used for treating or controlling the growth of the cancer.

The present pharmaceutical composition can be provided in a variety of dosage forms such as, by way of example and without limitation, solution, suspension, cream, ointment, lotion, capsule, tablet, caplet, gelcap, suppository, enema, transdermal patch, implant, gel, injectable, i.v. infusion bag or bottle, concentrate, dressing, elixir, syrup, emulsion, film, granule, gum, insert, jelly, foam, paste, pastille, pellet, spray, swab, tape, troche, lozenge, disk, magma, poultice, and-wafer.

Methods for the preparation of the dosage forms contemplated herein are described in the included examples or in the cited references, the disclosures of which are hereby incorporated herein in their entirety. Any ingredients used in the present formulation should not degrade or decompose a significant portion of the DFMO or other therapeutic compound(s) used prior to administration.

The term "unit dosage form" is used herein to mean a single or multiple dose form containing a quantity of the formulation, said quantity being such that one or more predetermined units are normally required for a single therapeutic administration. In the case of multiple dose forms, such as scored tablets, said predetermined unit will be one fraction, such as a half or quarter of a scored tablet, of the multiple dose form.

It is contemplated that a combination of rapid-acting, short-acting, fast-releasing, long-acting, colorectal release, sustained release, controlled release, pulsatile release, gastric release, enteric release, extended release, timed release or slow release dosage forms may be used in the present invention.

### Pharmaceutical Formulation and Administration

For injection, the pharmaceutical composition can be formulated, for reconstitution with an appropriate solution, as, for example and without limitation: freeze dried, rotary dried or spray dried powders; amorphous powders; or granules, precipitates or particulates. For injection, the composition may also be formulated as suspensions or liquids in the appropriate solutions, such as, by way of example and without limitation, water, aqueous solvents, nonprotic solvents, protic solvents, hydrophilic solvents, hydrophobic solvents, polar solvents, nonpolar solvent and/or combinations thereof, optionally containing stabilizers, pH modifiers, surfactants, bioavailability modifiers and/or combinations thereof. The pharmaceutical composition can be administered in the form of a depot injection or implant preparation which may be formulated in such a manner as to permit a sustained release of the active ingredient. The composition can be compressed into pellets or small cylinders and implanted subcutaneously or intramuscularly as depot injections or implants. Implants can employ inert materials such as biodegradable polymers or synthetic silicones, for examples, SILASTIC™, silicone rubber-manufactured by the Dow-Corning Corporation.

For oral, buccal, and sublingual administration, the pharmaceutical composition of the invention may be administered as either solutions or suspensions in the form of gelcaps, caplets, tablets, capsules or powders. For rectal administration, the compounds of the invention may be administered in the form of suppositories, ointments, enemas, tablets and creams for release of compound in the intestines, sigmoid flexure and/or rectum. It is contemplated that the pharmaceutical formulation can be formulated as, for example and without limitation, freeze dried, rotary dried or spray dried powders; amorphous or crystalline powders; or granules, precipitates or particulates. The solids used can be either free-flowing or compressed. The pharmaceutical formulation can comprise by way of example and without limitation, water, aqueous solvents, nonprotic solvents, protic solvents, hydrophilic solvents, hydrophobic solvents, polar solvents, nonpolar solvent, emollients and/or combinations thereof, optionally containing stabilizers, pH modifiers, surfactants, perfumes, astringents, cosmetic foundations, pigments, dyes, bioavailability modifiers and/or combinations thereof.

The pharmaceutical composition can also be administered as liquid suspensions or solutions using a sterile liquid, such as an oil, water, an alcohol, or mixtures thereof, with or without the addition of a pharmaceutically suitable surfactants, suspending agent, or emulsifying agent for oral or parenteral administration.

For liquid preparations, the pharmaceutical composition can be formulated suitably with oils, for example, fixed oils, such as peanut oil, sesame oil, cottonseed oil, corn oil and olive oil; fatty acids, such as oleic acid, stearic acid and isotearic acid; and fatty acid esters, such as ethyl oleate, isopropyl myristate, fatty acid glycerides and acetylated fatty acid glycerides; with alcohols, such as ethanol, isopropanol, hexadecyl alcohol, glycerol and propylene glycol; with glycerol ketals, such as 2,2-dimethyl-1,3-dioxolane-4-methanol; with ethers, such as poly(ethyleneglycol) 450, with petroleum hydrocarbons, such as mineral oil and petrolatum; with water, or with mixtures thereof; with or without the addition of a pharmaceutically suitable surfactant, suspending agent or emulsifying agent.

The solid unit dosage form of the invention will comprise DFMO and can be combined with conventional carriers, for example, binders, such as acacia, corn starch or gelatin; disintegrating agents, such as, corn starch, guar gum, potato starch or alginic acid; lubricants, such as, stearic acid or magnesium stearate; and inert fillers, such as lactose, sucrose or corn starch. The solid dosage form may comprise granules. As used herein, the term "granule" is taken to mean particle, crystal, powder, particulate, minitablet, compact or other similar solid forms. The granules used in the invention may display diffusion and/or dissolution controlled release rate profiles according to the components from and processes by which they are made.

For gelcap preparations, the pharmaceutical formulation may include oils, for example, fixed oils. such as peanut oil, sesame oil, cottonseed oil, corn oil and olive oil; fatty acids, such as oleic acid, stearic acid and isostearic acid; and fatty acid esters, such as ethyl oleate, isopropyl myristate, fatty acid glycerides and acetylated fatty acid glycerides; with alcohols, such as ethanol, isopropanol, hexadecyl alcohol, glycerol and propylene glycol; with glycerol ketals, such as 2,2-dimethyl-1,3-dioxolane-4-methanol; with ethers, such as poly(ethylene glycol) 450, with petroleum hydrocarbons, such as mineral oil and petrolatum; with water, or with mixtures thereof; with or without the addition of a pharmaceutically suitable surfactant, suspending agent or emulsifying agent.

Oils can also be employed in the preparation of formulations of the soft gelatin type. Water. saline, aqueous dextrose and related sugar solutions, and glycerols may be employed in the preparation of suspension formulations which may suitably contain suspending agents, such as pectin, carbomers, methyl cellulose, hydroxypropyl cellulose or carboxymethyl cellulose, as well as buffers and preservatives. Soaps and synthetic detergents may be employed as surfactants and as vehicles for detergent compositions. Suitable soaps include fatty acid alkali metal, ammonium, and triethanolamine salts. Suitable detergents include cationic detergents, for example, dimethyl dialkyl ammonium halides, alkyl pyridinium halides, and alkylamine acetates; anionic detergents, for example, alkyl, aryl and olefin sulfonates, alkyl, olefin, ether and monoglyceride sulfates, and sulfosuccinates; nonionic detergents, for example, fatty amine oxides, fatty acid alkanolamides, and poly(oxyethylene)-*block*-poly(oxypropylene) copolymers; and amphoteric detergents, for example, alkyl ,β-aminopropionates and 2-alkylimidazoline quaternary ammonium salts; and mixtures thereof.

The formulation may also comprise adsorbents, antioxidants, buffering agents, colorants, flavorants, sweetening agents, tablet antiadherents, tablet binders, tablet and capsule diluents, tablet direct compression excipients, tablet disintegrants, tablet glidants, tablet lubricants, tablet or capsule opaquants and/or tablet polishing agents.

As used herein, the term "adsorbent" is intended to mean an agent capable of holding other molecules onto its surface by physical or chemical (chemisorption) means. Such compounds include, by way of example and without limitation, powdered and activated charcoal and the like.

As used herein, the term "antioxidant" is intended to mean an agent which inhibits oxidation and thus is used to prevent the deterioration of preparations by the oxidative process. Such compounds include, by way of example and without limitation, ascorbic acid, ascorbyl palmitate, butylated hydroxyanisole, butylated hydroxytoluene, hypophophorous acid, monothioglycerol, propyl gallate, sodium ascorbate, sodium bisulfite, sodium formaldehyde sulfoxylate and sodium metabisulfite and the like.

As used herein, the term "buffering agent" is intended to mean a compound used to resist change in pH upon dilution or addition of acid or alkali. Such compounds include, by way of example and without limitation, potassium metaphosphate, potassium phosphate, monobasic sodium acetate and sodium citrate anhydrous and dihydrate and the like.

As used herein, the term "colorant" is intended to mean a compound used to impart color to liquid and solid (e.g., tablets and capsules) pharmaceutical preparations. Such compounds include, by way of example and without limitation, FD&C Red No. 3, FD&C Red No. 20, FD&C Yellow No. 6, FD&C Blue No. 2, D&C Green No. 5, D&C Orange No. 5, D&C Red No. 8, caramel, and ferric oxide, red and the like.

As used herein, the term "flavorant" is intended to mean a compound used to impart a pleasant flavor and often odor to a pharmaceutical preparation. In addition to the natural flavorants, many synthetic flavorants are also used. Such compounds include; by way of example and without limitation, anise oil, cinnamon oil, cocoa, menthol, orange oil, peppermint oil and vanillin and the like.

As used herein, the term "sweetening agent" is intended to mean a compound used to impart sweetness to a preparation. Such compounds include, by way of example and without limitation, aspartame, dextrose, glycerin, mannitol, saccharin sodium, sorbitol and sucrose and the like.

As used herein, the term "tablet antiadherents" is intended to mean agents which prevent the sticking of table formulation ingredients to punches and dies in a tableting machine during production. Such compounds include, by way of example and without limitation, magnesium stearate and talc and the like.

As used herein, the term "tablet binders" is intended to mean substances used to cause adhesion of powder particles in table granulations. Such compounds include, by way of example and without limitation, acacia, alginic acid, carboxymethylcellulose sodium, compressible sugar ethylcellulose, gelatin, liquid glucose, methylcellulose, povidone and pregelatinized starch and the like.

As used herein, the term "tablet and capsule diluent" is intended to mean inert substances used as fillers to create the desired bulk, flow properties, and compression characteristics in the preparation of tablets and capsules. Such compounds include, by way of example and without limitation, dibasic calcium phosphate, kaolin, lactose, mannitol, microcrystalline cellulose, powdered cellulose, precipitated calcium carbonate, sorbitol, and starch and the like.

As used herein, the term "tablet direct compression excipient" is intended to mean a compound used in direct compression tablet formulations. Such compounds include, by way of example and without limitation, dibasic calcium phosphate (e.g., Ditab) and the like.

As used herein, the term "tablet disintegrant" is intended to mean a compound used in solid dosage forms to promote the disruption of the solid mass into smaller particles which are more readily dispersed or dissolved. Such compounds include, by way of example and without limitation, alginic acid, carboxymethylcellulose calcium, microcrystalline cellulose (e.g., AVICEL), polacrilin potassium (e.g., AMBERLITE), sodium alginate, sodium starch glycolate, and starch and the like.

As used herein, the term "tablet glidant" is intended to mean agents used in tablet and capsule formulations to reduce friction during tablet compression. Such compounds include, by way of example and without limitation, colloidal silica, cornstarch, and talc and the like.

As used herein, the term "tablet lubricant" is intended to mean substances used in tablet formulations to reduce friction during tablet compression. Such compounds include, by way of example and without limitation, calcium stearate, magnesium stearate, mineral oil, stearic acid, and zinc stearate and the like.

As used herein, the term "tablet/capsule opaquant" is intended to mean a compound used to render a capsule or a tablet coating opaque. May be used alone or in combination with a colorant. Such compounds include, by way of example and without limitation, titanium dioxide and the like.

As used herein, the term "tablet polishing agent" is intended to mean a compound used to impart an attractive sheen to coated tablets. Such compounds include, by way of example and without limitation, carnauba wax, and white wax and the like.

It should be understood, that compounds used in the art of pharmaceutical formulation generally serve a variety of functions or purposes. Thus, if a compound named herein is mentioned only once or is used to define more than one term herein, its purpose or function should not be construed as being limited solely to that (those) named purpose(s) or function(s).

The course and duration of administration of and the dosage requirements for the formulation of the present invention will vary according to the subject being treated, the formulation used, the method of administration used, the severity and type of estrogen independent breast cancer or tumor being treated, the coadministration of other drugs and other factors.

Although each unit dosage form contains therapeutically effective amounts of DFMO, it may be necessary to administer more than one such unit dosage form in order to obtain the full therapeutic benefit of the DFMO. More particularly, since DFMO may require moderately high doses, *vide supra,* it is very likely that more than one unit dosage from will need to be administered to a patient in order to obtain the full therapeutic benefit of DFMO.

For example, consider that the average 70 Kg man has a body surface area of 1.73 m². If DFMO is administered at a dosage of up to about 3 g/m²/day, then a patient would have to receive about 5 g of DFMO/day, about 10 tablets containing 0.5 g of DFMO. Correspondingly, if the dosage administered is about 0.25 g/m²May then a patient would receive about 0.4 g/day, about I tablet containing 0.5 g of DFMO.

As used herein, the term DFMO is intended to mean a preparation of alpha-difluoromethylornithine in its pharmaceutically acceptable salt and/or isomeric forms. (+)-DFMO is intended to mean alpha-difluoromethylornithine having the (D)-configuration around the alpha-carbon which is the only chiral atom present in the molecule. (-)-DFMO is intended to mean alpha-difluoromethylornithine having the (L)- configuration around the alpha-carbon. (+/-)-DFMO is intended to mean racemic alpha-difluoromethylornithine.

Methods for the preparation of (+)-DFMO and (-)-DFMO are known. U.S. 4,330,559, the disclosure of which is hereby incorporated by reference in its entirety, discloses a method for the preparation of optically pure DFMO wherein racemic DFMO dihydrochloride is reacted with sodium methylate to form 3-amino-3-difluoromethyl-2-piperidone (DFMO-pip) which is subsequently crystallized in the presence of (-)-binaphthyl phosphoric acid ((-)-BNPA) to yield (-)-DFMO-pip:(-)-BNPA 1:1 addition salt crystals leaving the (+)-DFMO-pip:(-)-BNPA addition salt in solution. Following repeated recrystallization and acidification, (-)-DFMO-pip is obtained in optically pure form. The (-)-DFMO-pip is then hydrolyzed to yield (+)-DFMO. The enantiopode (+)-DFMO may be prepared according to the above procedure by employing (+)-BNPA to preferentially form the diastereomeric (+)-DFMO-pip:(+)-BNPA 1:1 addition salt crystals.

Wagner, et al. (1987), the disclosure of which is hereby incorporated by reference in its entirety, discloses a reverse phase liquid chromatographic method for the resolution of racemic DFMO to yield each enantiomer of DFMO in optically pure form.

Aldous et al. (1986) discloses a gas chromatographic analytical method for the resolution of racemic DFMO to yield each enantiomer of DFMO in optically pure form.

The MDA-MB-23 1 human breast tumor cell line is estrogen independent. The cell line cannot be maintained in vitro and so was maintained in vivo in host nude mice. The tumor growth inhibition studies were conducted by transplanting MDA-MB-231 tumor cells from the host nude mouse to the subject and control nude mice of the study. The MDA-MB-231 cell line can be obtained from the American Type Culture Collection as ATCC HTB 26.

As used herein when referring to taxol, the term "derivative of" means a prodrug or structurally modified analog of taxol or any of the species in the class of compounds known as taxines such as, by way of example and without limitation, a metabolically cleavable ester or ether of taxol, an optical or diastereomeric form of taxol or taxine A and the like. As used herein when referring to taxol, the term "metabolite of" means a compound formed directly or indirectly as a result of a metabolic pathway, biotransformation or chemical transformation in a patient.

The compounds herein described may have asymmetric centers. All chiral, diastereomeric, and racemic forms are included in the present invention. Many geometric isomers of olefins, C=N double bonds, and the like can also be present in the compounds described herein, and all such stable isomers are contemplated in the present invention unless the specific stereochemistry or isomer form is specifically indicated. It will be appreciated that certain compounds of the present invention contain an asymmetrically substituted carbon atom, and may be isolated in optically active or racemic forms. It is well known in the art how to prepare optically active forms, such as by resolution of racemic forms or by synthesis, from optically active starting materials. Also, it is realized that cis and trans geometric isomers of the compounds of the present invention are described and may be isolated as a mixture of isomers or as separated isomeric forms.

The following abbreviations are used herein and are defined as follows:
- DFMO: alpha-difluoromethylornithine
- ODC: ornithine decarboxylase
- RA: retinoic acid
- ER: estrogen receptor
- IGF- 1: insulin-like growth factor 1
- TGF-beta: transforming growth factor beta
- MX: methotrexate
- FU: 5-fluorouracil
- TC: tamoxifen citrate
- TAM: tamoxifen
- DX: doxorubicin
- VS: vincristine sulfate
- MNU: 1-methyl-1-nitrosourea
- E2: estradiol
- CIN III: cervical intrapithelial neoplasia grade 3
- TGI: tumor growth inhibition
- DFMO-pip: 3-amino-3-difluoromethyl-2-pipenidone
- BNPA: binaphthyl phosphoric acid
- MTD: maximum tolerated dose

### DESCRIPTION OF THE PREFERRED EMBODIMENTS

Following long-standing patent law convention, the terms "a" and "an" mean "one or more" when used in this specification.

One embodiment of the invention provides a pharmaceutical composition for use as a medicament, in particular for use in treating, preventing, controlling the growth of and/or reducing the risk of estrogen independent breast cancer and solid tumor comprising taxol, or a prodrug, derivative or metabolite thereof, and (+)-DFMO, (-)-DFMO or a defined ratio of (+)-DFMO: (-)-DFMO or pharmaceutically acceptable salts thereof present in the amount of about 0.01% to 90% by weight of the composition and a pharmaceutical carrier.

Another embodiment of the invention provides the use of a therapeutically effective amount of taxol, or a derivative, prodrug or metabolite thereof, and (+)-DFMO, (-)-DFMO or a defined ratio of (+)-DFMO: (-)-DFMO, or pharmaceutically acceptable salts thereof for the manufacture of a medicament for treating, preventing, controlling the growth of and/or reducing the risk of developing estrogen independent breast cancer and tumor.

Another embodiment of the invention provides the use of a therapeutically effective amount of taxol and ( +)-DFMO, (-)-DFMO or a defined ratio of (+)-DFMO: (-)DFMO or pharmaceutically acceptable salts thereof for the manufacture of a medicament for treating and controlling the growth of estrogen independent breast cancer and tumor.

Another embodiment of the invention provides the use of therapeutically effective amounts of taxol and (+)-DFMO, (-)-DFMO or a mixture of (+)-DFMO: (-)-DFMO, or pharmaceutically acceptable salts thereof for the manufacture of a medicament for treating and reducing the risk of developing estrogen independent breast cancer and inhibiting estrogen independent cancer cell growth.

Unless otherwise indicated, all chemicals were purchased from Aldrich Chemicals (Milwaukee, WI). Racemic DFMO is available from Ilex Oncology, Inc. (San Antonio, TX).

### EXAMPLE 1

### PREPARATION OF (+)- and (-)-DFMO BY CRYSTALLIZATION

**3-Amino-3-difluoromethyl-2-piperidone:** To a solution of methyl-2-difluoromethyl-2,5-diaminopentanoate-dihydrochloride (2.7 g) in dry methanol (30 ml) is added under nitrogen 2 equivalents of sodium methylate in methanol (0.46 g of sodium in 20 ml of methanol). The reaction mixture is stirred for 3 hours at room temperature then the solvent is evaporated under reduced pressure. The residue is extracted with ether to yield crude 3-amino-3-difluoromethyl-2-piperidone which is purified either by crystallization from CHCl₃/pentane: (m.p.: 149°C.) or by distillation (b.p. 135°C./0.05 mmHg).

**(-)- and (+) 3-Amino-3-difluoromethyl-2-piperidine hydrochloride:** To a solution of (―)-binaphthylphosphoric acid ((-)-BNPA) (1.27 g) in hot ethanol (50 ml) is added a solution of (±)-3-amino-3-difluoromethyl-2-piperidone (0.546 mg) in hot ethanol (5 ml). On cooling, crystals separate. The reaction mixture is then let stand at 4°C. overnight. The precipitate is filtered off, washed with ethanol and diethyl ether to give 0.54 g of (-)-binaphthylphosphoric salt ([α]^{t}_{D}=―409°C. =0.3, MeOH mp: 300°C.). Recrystallization of the mother liquor yields 0.15 g of (―)-binaphthylphosphoric salt. Concentration of the filtrate gives 1.1 g of a sticky material which is treated with HCl 3 M at room temperature for 3 hours. The (―)-BNPA is filtered off and the filtrate concentrated under reduced pressure. Recrystallization of the residue (320 mg) in ethanol affords (+)-3-amino-3-difluoromethyl-2-piperidonemonohydrochloride (160 mg) ([α]^{t}_{D}=+18°6, C=1, MeOH) m.p.: 238°C.). Treated in the same condition the (―)-BNPA salt (436 mg) gives (-)-3-amino-3-difluoromethyl-2-piperidonemonohydrochloride (137 mg) which is crystallized in ethanol (67 mg) ([α]^{t}_{D}=―19°, C = 1.02, MeOH; mp =240°C.dec.).

**(―)- and (+)-2-difluoromethyl-2,5-diamino pentanoic acid monohydrochloride:**
(-)-3-Difluoromethyl-3-amino-2piperidonehydrochloride (60 mg) is heated in HCl 6 M (4 ml) at reflux for 12 hours. After concentration under reduced pressure, the residue is dissolved in water and the pH of the solution is adjusted to 4.5 with a solution of NEt₃. The solution is then concentrated under reduced pressure and the residue extracted many times with chloroform and then recrystallized from H₂O/EtOH to give (+)-2-difluoromethyl-2,5-diaminopentanoic acid monohydrochloride (54 mg) ([α]_{ρ}=+6°, C=0,48, MeOH; mp≥240°C.). By an identical treatment, (+)-3-difluoromethyl-3-amino-2-piperidone hydrochloride (96 mg) gives (-)-2-difluoromethyl-2,5-diaminopentanoic acid monohydrochloride (56 mg)[α]^{t}_{D}=-10°, C=0.7, MeOH, mp≥244°).

### EXAMPLE 2

### PREPARATION OF (-)-DFMO BY ENZYMATIC RESOLUTION

The following method is the same as that disclosed by Au et al. (1990), the disclosure of which is hereby incorporated in its entirety.

Racemic 3-amino-3-difluoromethy-2-piperidone (250 mg) is treated with L-alpha-amino-ε-caprolactam hydrolase in Tris-HCl buffer (0.2M, pH 8.5) containing MnCl₂ (1 M). The mixture is stirred for 48 h and then extracted with chloroform. The aqueous layer is separated, acidified with 6N HCl and concentrated to dryness. The residue is recrystallized from a water-ethanol mixture to yield (-)-DFMO (36 mg).

### EXAMPLE 3

### DETERMINATION OF (+)- AND (-)-DFMO IN BODY FLUIDS

**Instrumentation:** A gas chromatograph (5730A, Hewlett Packard, Pittsburgh, PA, USA), equipped with an electron capture detector (ECD with pulsed variable frequency and ⁶³Ni as radioactive source) and a recorder-integrator (Hewlett Packard 3380A) is employed. The sample injection is made (Hewlett Packard 7672A autosampler) in the splitness mode (purge delay 30 sec.) on a Chirasil-L-Val capillary fused silica column (Chrompack: WCOT 25 m x 0.22 mm ID, film thickness 0.12 µm). The elusion of the two enantimers of αDFMO and the internal standard (IS is MFMO:(+),(-)-α-monofluoromethylornithine:MDL 71.919) is realized with argon-methane 5% (quality HP) as carrier gas (column pressure 0.6 atm) and with a temperature gradient ranging from 140° to 190°C at a rate of 2°C/min, following a 2 min isothermal period. The injection portion has a temperature of 250°C and the detector temperature is kept at 350°C.

**Plasma samples:** To 100 µl of human blank plasma is added known quantities of(+)-aDFMO (MDL 71.872) or (-)-αDFMO (MLD 71.871) and (+, -)-MFMO (IS). Plasma proteins are precipitated with 400 µl of methanol and the mixture is vigorously shaken. The sample is centrifuged for 15 min at 5000 rpm (Sorval®Instrument GLC 4-Dupont). The supernatant is transferred to another vial and evaporated to dryness with a stream of nitrogen. One hundred µl of HBF and 50 µl of HFBA are added to the residue, and the reaction mixture is allowed to stand for 1 h at room temperature. After evaporation of the volatile reagents under nitrogen, the residue is partitioned between dichloromethane (4 ml) and 0.1 M sodium phosphate buffer ph = 5.8 (1 ml). The organic layer is isolated after centrifugation for 10 min at 3000 rpm and further washed with 1 ml of phosphate buffer pH = 5.8. The final organic phase is evaporated to dryness and reconstituted with 400 µl of ethyl acetate and transferred into autosampler vials. One µI of this solution is then injected onto the GC column.

**Urine samples:** a 100 µl aliquot of 24 h human blank urine is directly evaporated under nitrogen after addition of (+) or (-)-αDFMO and the IS is known quantities. In the same way as with plasma, 100 µl of HFB and 50 µl of HFBA are added and the mixture is allowed to react at room temperature for 1 h. After evaporation of the excess of the reagents, the residue is partitioned between 4 ml of dichloromethane and 1 ml of 0.1 M sodium phosphate buffer pH = 5.8. To eliminate interfering acidic constituents of the urine, the organic layer is further washed with 5 mM Tris buffer (pH = 8.6). After evaporation, the residue of the organic layer is reconstituted with 400 µl of ethyl acetate. One µl of this solution is injected onto the column.

### EXAMPLE 4

### DETERMINATION OF TUMOR GROWTH INHIBITION OF MDA-MB-231 BREAST TUMOR BY DFMO AND/OR TAXOL

Nude mice are implanted s.c. by trocar with fragments of MDA-MB-231 human breast tumor cells harvested from s.c. growing tumors in nude mice hosts. When tumors are approximately 5 mm x 5 mm in size (usually about ten to twenty days after inoculation), the animals are pair-matched into treatment and control groups. Each group contains 10 tumored mice, each of which is ear-tagged and followed individually throughout the experiment. The administration of drugs or vehicle begins the day the animals are pair-matched (Day 1). The doses, route of drug administration and schedule are selected as appropriate for the study in question. If the MTD dose of an agent is not known, it is determined in an initial dosing experiment in non-tumored mice.

The experiment is usually terminated when control tumors reach a size of 1 g or 2 g for the tumor models. Mice are weighed twice weekly, and tumor measurements are taken by calipers twice weekly, starting on day 1. These tumor measurements are converted to mg tumor weight by a well-known formula, and from these calculated tumor weights, the termination date can be determined. Upon termination, all mice are weighed, sacrificed, and their tumors excised. Tumors are weighed, and the mean tumor weight per group is calculated. In this model, the mean treated tumor weight/mean control tumor weight x 100% (T/C) is subtracted from 100% to give the tumor growth inhibition (TGI) for each group.

The final weight of a given tumor is subtracted from its own weight at the start of treatment on day 1. This difference divided by the initial tumor weight is the % shrinkage. A mean % tumor shrinkage can be calculated from data from the mice in a group that experienced regressions. If the tumor completely disappears in a mouse, this is considered a complete regression or complete tumor shrinkage. If desired, mice with partial or total tumor regressions can be kept alive past the termination date to see whether they live to become long term, tumor-free survivors. Statistics are performed on the data using primarily the log rank-value test.

In the above procedure, a reduction in the growth, number, size, metastasis and/or reoccurrence of tumor in the animal is deemed a positive response.

The results for this assay are shown in Figure 2 and summarized in Table 1.

| Group | n | Dose & Route | Schedule | Final Tumor Wt. (Mean±SEM) | % Tumor Growth Inhibition |
|---|---|---|---|---|---|
| Control | (9) | Vehicle/i.p | Qdx5 | 1565.4±351.3 | 0 |
| DFMO | (9) | 0.5%/p.o. | ad libitum | 1276.3±398.6 | 19.2 |
| DFMO | (9) | 3%/p.o. | ad libitum | 782.7±111.4 | 51.8 |
| Taxol | (9) | 20mg/kg/i.p. | qdx5 | 301.6±72.9 | 83.7 |
| DFMO+ Taxol | (9) | 0.5%/p.o. 20mg/kg/i.p. | ad libitum qdx5 | 212.9±30.9 | 89.6 |
| Taxol | (9) | 3%/p.o. 20mg/kg/i.p. | ad libitum qdx5 | A.D. | A.D. |
| *Taxol was administered on Day 8. A.D. indicates all animals died. | | | | | |

### EXAMPLE 5

### PREPARATION OF (+)-DFMO AND (-)-DFMO-CONTAINING TABLE I COMPRISING RAPID AND SLOW RELEASE LAYERS

One thousand layered tablets, comprising a slow release layer and a rapid release layer of α-difluoromethylornithine are prepared as follows:

### Slow Release Layer

(a) (-)-α-Difluoromethylornithine: 300.0 gm
(b) Hydroxypropyl methylcellulose (400 cps): 100.0 gm
(c) Mannitol: 100.0 gm
(d) Corn starch: 6.0 gm
(e) Zinc stearate: 3.6 gm

### Rapid Release Layer

(f) (+)-α-Difluoromethylornithine: 500.0 gm
(g) Microcrystalline cellulose: 100.0 gm
(h) Starch: 100.0 gm

Using a suitable mixer, the (-)-α-difluoromethylomithine, mannitol and hydroxypropyl methylcellulose are mixed well via geometric dilution. The mixture is mixed in a Fitzmill quipped with a No. 000 screen and granulated using a 5% starch paste prepared by adding the corn starch to approximately 115 ml of water. Additional water is added as required to make a suitable granulation. The resulting granulation is wet-screened using a No. 2 screen and tray dried at 40°C. to 50°C. for 8 to 12 hours. The dried granulation is ground and passed through a No. 10 screen. Zinc stearate, which has passed through a No. 20 screen is added to the granulation, mixed well and the resulting slow release granulation reserved for tablet compression.

The (+)-α-difluoromethylomithine for the rapid release layer is milled, if necessary, to obtain a powder having the majority of particles in the range of 10 to 150 microns in size. The milled powder, microcrystalline cellulose and starch are mixed well in a Fitzmill equipped with No. 000 screen and the resulting rapid release mixture reserved for tablet compression.

Using a suitable layer press, such as the Manesty Layer Press, the slow release granulation is added to the adjusted die cavity to provide a layer having a weight of approximately 500 mg. The rapid release granulation is added to the die cavity and the final compression pressure is adjusted to provide a suitable tablet with a total weight of approximately 1.2 g.

### EXAMPLE 6

### DETERMINATION OF TUMOR GROWTH INHIBITION OF MCF-7 BY DFMO AND/OR TAXOL

Female ovariectomized nude mice are implanted s.c. by trocar with 21 day release 0.25 mg estrogen pellets. The following day the mice are implanted s.c. by trocar fragments of MCF-7 mammary carcinomas harvested from s.c. growing MCF-7 tumors in nude mice hosts. When tumors are approximately 5 mm x 5 mm in size (about twenty days after inoculation), the animals are pair-matched into treatment and control groups, and the estrogen pellets removed. Each group contains 10 tumored mice, each of which is ear-tagged and followed individually throughout the experiment. The administration of drugs or vehicle begins the day the animals are pair-matched (Day 1). The doses, route of drug administration and schedule are selected as appropriate for the study in question. If the MTD dose of an agent is not known, it is determined in an initial dosing experiment in non-tumored mice.

Mice are weighed twice weekly, and tumor measurements are taken by calipers twice weekly, starting on Day 1. These tumor measurements are converted to mg tumor weight by a well-known formula, L²xW/2. The experiment is terminated when control tumors reach a size of 1 gram. Upon termination, all mice are weighed, sacrificed, and their tumors excised. Tumors are weighed and the mean tumor weight per group is calculated. In these models, the mean treated tumor weight/mean control tumor weight x 100% (T/C) is subtracted from 100% to give the tumor growth inhibition (TGI) for each group.

The final weight of a given tumor is subtracted from its own weight at the start of treatment on Day 1. This difference divided by the initial tumor weight is the % shrinkage. A mean % tumor shrinkage can be calculated from data from the mice in a group that experienced tumor regressions. If the MCF-7 tumor completely disappears in a mouse, this is considered a complete regression or complete tumor shrinkage. If desired, mice with partial or total tumor regressions can be kept alive past the termination date to see whether they live to become long term, tumor-free survivors.
Results for this assay are shown in Figure 1 and summarized in Table 2 below.

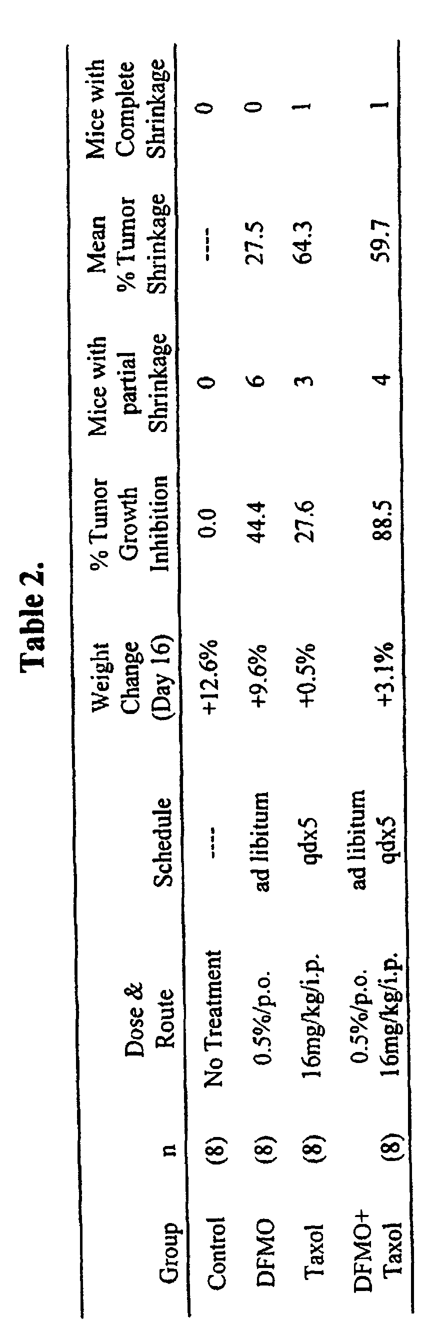

### EXAMPLE 7

### PREPARATION OF (-)-DFMO-CONTAINING TOPICAL SOLUTION

Ethyl alcohol (8 mL) is thoroughly mixed with isopropyl myristate (5 g) and poly(ethylene glycol) 400 (10 g). While mixing add (-)-DFMO (8.5 g) and sufficient purified water to make a total 100 mL volume.

### EXAMPLE 8

### PREPARATION OF (-)-DFMO-CONTAINING LOTION

Isostearic acid (10 g) and stearic acid (8 g) are thoroughly mixed with poloxamer 235 (10 g). While mixing, add to this mixture propylene glycol (10 g) and (-)-DFMO (10 g) until thoroughly mixed. Finally, add with mixing sufficient purified water to make a 100 mL formulation final volume.

### EXAMPLE 9

### PREPARATION OF (+)-DFMO-CONTAINING TOPICAL SOLUTION

A vehicle (100 g) is prepared by mixing the following ingredients in the specified amounts (weight percent based upon the total vehicle weight): water (68 %), ethanol (16 %), propylene glycol (5 %), dipropylene glycol (5 %), benzyl alcohol (4%) and propylene carbonate (2 %). Varying amounts of the vehicle (90 to 99.5 g) are then thoroughly mixed with (+)-DFMO (10 to 0.5 g).

### EXAMPLE 10

### PREPARATION OF (+)-DFMO-CONTAINING GRANULES FOR DISSOLUTION IN WATER

(+)-DFMO (33 g) is mixed with lactose (6 g) and passed through a fluid energy mill or micronizer to give a particle size of 1-25 microns. Water (35 mL) is added to corn starch (2 g) and blended to prepare a starch paste. The micronized (+)-DFMO-lactose powder, lactose (42.2 g) and corn starch (16.5 g) are well blended. The starch paste is added and the entire mixture blended. The resulting mixture is passed through as No. 12 mesh screen. The resulting granules are dried at 38 C to a moisture content of about 3 % by weight, ground thorough a U.S. Standard No. 12 screen and lubricated by mixing with 0.3 g or zinc stearate. The above formulation is suitable for dissolution in water for oral administration.

### EXAMPLE 11

### PREPARATION C)F (-)-DFMO-CONTAINING RECONSTITUTABLE DRY BEVERAGE BASE

The following ingredients are thoroughly mixed in the amounts specified: Fries & Fries grapefruit flavoring #91470 (5.0 g), fructose USP (30.0 g), aspartame (0.5 g), citric acid (anhydrous, 2.0 g) and (-)-DFMO (30.0 g). The above mixture will generally be administered to a patient by reconstituting 10 % by weight of the final formulation in water (200 mL) to arrive at a unit dose for oral administration as for the treatment or prevention of breast cancer.

### EXAMPLE 12

### PREPARATION OF (-)-DFMO-CONTAINING ORAL SOLUTION

To distilled water (7.0 mL) is added sodium benzoate (15 mg), and saccharin sodium (18 mg) and the mixture heated to 50 - 60°C. (-)-DFMO (3.0 g) is added until dissolution. After cooling the solution to 20 - 30°C, add ethanol 0.75 mL, glycerin (0.75 mL), propylene glycol (1.5 mL) and water to a final solution volume of 15 mL. The above formulation is suitable for oral administration as for the treatment or prevention of breast cancer.

### EXAMPLE 13

### PREPARATION OF (-)-DFMO-CONTAINING CONVENTIONAL RELEASE HARD GELATIN CAPSULES

One thousand two-piece hard gelatin capsules for oral use, each containing 200 mg. of (-)-DEMO are prepared from the following types and amounts of materials:
(-)-DFMO: 200 gm.
Corn starch: 150 gm.
Talc: 75 gm.
Magnesium stearate: 2.5 gm.

The materials are thoroughly mixed and then encapsulated in the usual manner.

Using the procedure above, capsules are similarly prepared containing in 5, 100, and 500 mg. amounts by substituting 5, 100, and 500 gm. of (-)-DFMO for the 200 gm. used above.

### EXAMPLE 14

### PREPARATION OF TAXOL AND (+)-DFMO-CONTAINING TOPICAL OINTMENT

(+)-DFMO: 50 gm
Taxol: 10 gm.
Liquid petrolatum (heavy): 250 gm.
Wool fat: 200 gm.
White petrolatum q.s.: 1000 gm.

The white petrolatum and wool fat are melted and 100 gm. of liquid petrolatum added thereto. The taxol and (+)-DFMO are added to the remaining liquid petroleum and the mixture milled until the powder is finely divided and uniformly dispersed. The powder mixture is stirred into the white petrolatum mixture and stirring continued until the ointment congeals.

### EXAMPLE 15

### PREPARATION OF TAXOL AND (-)-DFMO-CONTAINING CREAM

A)
   (-)-DFMO: 1000 gm.
   taxol: 50 gm.
   Cetyl alcohol: 600 gm.
   Stearyl alcohol: 600 gm.
   Aerosol OT: 150g.
   White petrolatum: 3000 gm.
   Propylene Glycol: 1000 ml.
   Distilled Water q.sl.: 10000 gm.

   The (-)-DFMO and taxol are mixed with the white petrolatum and stirred into a melt of the alcohols and propylene glycol. The aerosol OT is dissolved in 5000 cc. of water and an emulsion formed with the petrolatum mix, sufficient water being added to make 10,000 gm.
   Optionally, substituting 2,000 grams of dimethylacetamide for 2000 grams of water, or 200-500 grams of dimethylsulfoxide for 200-500 grams of water, a composition is obtained providing better penetration of the active ingredients into the skin.
B) 1000 grams of a topical cream is usefully prepared from the following types and amounts of ingredients:
   (-)-DFMO: 100 grams
   Taxol: 10 grams
   Tegacid Regular: 150 grams
   Spermaceti: 100 grams
   Propylene glycol: 50 gm.
   Polysorbate 80: 5 gm.
   Methylparaben: 1 g. Deionized water q.s.: 1000 gm.

The (-)-DFMO and taxol are added to the other components in the same manner described above. This composition can be applied topically with occlusive bandage for treating, preventing and controlling the growth of estrogen independent breast cancer.

### EXAMPLE 16

### PREPARATION OF (-)-DFMO-CONTAINING CONVENTIONAL RELEASE TABLETS

A) One thousand tablets suitable for oral use prepared in accordance with the following formulation:
   (a) (-)-α-Difluoromethylornithine: 500.0 gm
   (b) Dicalcium phosphate: 250.0 gm
   (c) Methylcellulose, U.S.P. (15 cps): 6.5 gm
   (d) Talc: 20.0 gm
   (e) Calcium stearate: 2.5 gm

   The (-)-α-difluoromethylornithine and dicalcium phosphate are mixed well as a dry powder. The resulting powder is granulated using a 7.5% aqueous solution of methylcellulose, passed through a No. 8 screen and carefully dried. The dried granules prepared in this fashion are passed through a No. 12 screen, lubricated with the remaining talc and calcium stearate, and compressed into tablets. Each tablet contains 500 mg of (-)-α-difluoromethylomithine and one or more of such tablets are suitable for oral administration b.i.d. to q.i.d.
B) Another illustrative composition for tablets is as follows:
   (a) (-)-DFMO: 200 mg
   (b) starch: 43 mg
   (c) lactose: 45 mg
   (d) magnesium stearate: 2 mg

The granulation obtained upon mixing the lactose with the compound (a) and part of the starch and granulated with starch paste is dried, screened, and mixed with magnesium stearate. The mixture is compressed into tablets weighing 290 mg each.

### EXAMPLE 17

### PREPARATION OF (-)-DFMO-CONTAINING ORAL SYRUP

One thousand cc. of an aqueous suspension for oral use, containing in each 5 cc. dose 200 mg. of (-)-DFMO is prepared from the following types and amounts of ingredients:
(-)-DFMO: 39 gm.
Taxol: 1 gm.
Citric acid: 2 gm.
Benzoic acid: 1 gm.
Sucrose: 700 gm.
Tragacanth: 5 gm.
Lemon oil: 2 cc.
Deionized water q.s.:

The citric acid, benzoic acid, sucrose, tragacanth, and lemon oil are dispersed in sufficient water to make 850 cc. of solution. The (-)-DFMO and taxol are stirred into the syrup until uniformly distributed. Sufficient water is added to make 1000 cc. The composition so prepared is useful in the systemic treatment of estrogen independent breast cancer.

### EXAMPLE 18

### PREPARATION OF (-)-DFMO-CONTAINING INJECTABLE SUSPENSION

An illustrative composition for an injectable suspension is the following 1 ml ampul for an intramuscular injection.
(a) (-)-2,5-diamino-2-difluoromethyl pentanoic acid: 20 wt. %
(b) polyvinylpyrrolidone: 0.5 wt. %
(c) lecithin: 0.25 wt. %
(d) water for injection to make: 100.0 wt. %

The materials (a)-(d) are mixed, homogenized, and filled into 1 ml ampule which are sealed and autoclaved 20 minutes at 121°C. Each ampul contains 200 mg per ml of compound (a).

### EXAMPLE 19

### PREPARATION OF (-)-DFMO-CONTAINING INJECTABLE SOLUTION

A sterile aqueous solution for intramuscular use, containing in 1 cc. 75 mg. of (-)-DFMO is prepared from the following types and amounts of materials:
(-)-DFMO: 73.5 gm.
Taxol: 1.5 gm.
Methylparaben: 2.5 gm.
Propylparaben: 0.17 gm.
Water for injection q.s.: 1000 cc.

The ingredients are dissolved in the water and the solution sterilized by filtration. The sterile solution is filled into vials and the vials sealed. The composition is useful in the systemic treatment of estrogen independent cancers and tumors.

The above is a detailed description of a particular embodiment of the invention. It is recognized that departures from the disclosed embodiment may be made within the scope of the invention and that obvious modifications will occur to a person skilled in the art. The full scope of the invention is set out in the claims that follow and their equivalents. Accordingly, the claims and specification should not be construed to unduly narrow the full scope of protection to which the invention is entitled.

Those of skill in the art should, in light of the present disclosure, appreciate that many changes can be made in the specific embodiments where are disclosed herein and still obtain a like or similar result without departing from the spirit and scope of the invention. All of the compositions and methods disclosed and claimed herein can be made and executed without undue experimentation in light of the present disclosure. It will be apparent that certain compounds which are both physiologically and chemically related may be substituted for the therapeutic compound described herein while the same or similar results are achieved.

### REFERENCES

The following references, to the extent that they provide exemplary procedural or other details supplementary to those set forth herein, are specifically incorporated herein by reference.
1. ALDOUS S, ET AL. Resolution of(+)- and (-1-alpha-difluoromethylornithine by capillary gas chromatography. J. Chromatogr. (1986) 357 (2) 335-9
2. ANSEL HC, POPOVICH NG AND ALLEN LV, JR, EDS. Pharmaceutical Dosage Forms and Drug Delivery Systems, 6th Ed. Williams & Wilkins, Baltimore, ISBN 0-683-00193-0, 1995
3. CARBONE PP, LOVE RR, CAREY P, TUTSCH K, VERMA AK, WILDING
4. G, GILMORE-CUNNINGHAM D. Difluoromethylornithine (DFMO), a Potential Chemopreventive (Meeting Abstract). Proc. Annual Meet Am Assoc Cancer Res. Vol. 32, pp. A1209, 1991.
5. COHEN FJ; MANNI A; GLIKMAN P; BARTHOLOMEW M; DEMERS L. Interactions between growth factor secretion and polyamines in MCF-7 breast cancer cells. European Journal of Cancer, (1990) 26 (5) 603-8.
6. CREAVEN PJ, PENDYALA L, PORTER CW, MURPHY, MJ. Alpha-Difluoromethylornithine (DFMO) as a Potential Chemopreventive Agent: Toxicology, Pharmacokinetics and Pharmacodynamics of Chronic Oral Administration in Humans (Meeting abstract); Non-serial, (1993). CCPC-93: Second International Cancer Chemo Prevention Conference. April 28-30, 1993, Berlin, Germany, p. 53.
7. CREAVEN PJ, PENDYALA L, PETRELLI N, DOUGLASS H, HERRERA L, PORTER C, SOLOMON J. Phase I Study of Difluoromethylornithine DFMO as a Chemopreventive Agent (CPA) (meeting abstract); Proc. Annual Meet Am. Soc. Clin. Oncol. Vol. 11, pp. A395, 1992.
8. CROGHAN MK, AICKIN MG, MEYSKENS FL JR Dose-related α-difluoromethylomithine ototoxicity. Am. J. Clin. Oncol. 14:331-335, 1991.
9. CROWELL JA, GOLDENTHAL EI, KELLOFF GJ, MALONE WF, BOONE. Chronic Toxicity Studies of the Potential Cancer Preventive 2-(difluoromethyl) d,l-ornithine. CW Fundam Appl Toxicol, (1994) 22/3 (341-354).
10. FONTANA J A; MEZU A B; COOPER B N; MIRANDA D. Retinoid Modulation of Estradiol-Stimulated Growth and of Protein Synthesis and Secretion in Human Breast Carcinoma Cells. Cancer Res. (1990) 50, (7), 1997-2002.
11. FONTANA JA, ET AL. Retinoic acid inhibition of human breast carcinoma proliferation is accompanied by inhibition of the synthesis of a M(r) 39,000 protein. Cancer Res. (1990), 50(7), 1977-1982.
12. GOLDENTHAL EI. *One Year Oral Toxicity Study of Difluoromethylornithine in Rats and in Dogs.* International Research and Development Corporation, Reports 560-032 and 560-033, 1990.
13. GREENWALD P, MALONE WF, CERNY ME, STERN HR. Cancer Prevention Research Trials; Maryland 20892, U.S.A. 1993. Adv. Cancer Res. (1993) (61, 1-23, 1993) 5 Fig. 4 Tab. 74 Ref.
14. GRIFFIN CA, SLAVIK M, CHIEN SC, HERMANN J, THOMPSON G, BLANC O. Phase I Trial and Pharmacokinetic Study of Intravenous and Oral Alpha-Difluoromethylornithine; Invest. New Drugs (1987) 5, No. 2, 177-86.
15. GRIFFIN C, ABELOFF MD, SLAVIK M, ET AL. Phase I trial and pharmacokinetic study of intravenous and high dose oral α-difluoromethylornithine (DFMO). Proc. ASCO 3 :34, 1984.
16. GRIFFIN CA, SLAVIK M, CHIEN SC, ET AL. Phase I trial and pharmacokinetic study of intravenous and oral a-difluoromethylomithine. Invest new Drugs 5:177-186, 1987.
17. HAEGELE KD, ALKIN RG. ET AL. Kinetics of alpha-difluoromethylornithine: an Irreversible Inhibitor of Ornithine Decarboxylase. Clin. Pharmacol Ther. 30(2):210-17, 1981.
18. HOGGARD N; GREEN C D. Polyamines and growth regulation of cultured human breast cancer cells by 17 beta-oestradiol. Molecular and Cellular Endocrinology, (1986 Jun) 46 (1) 71-8.
19. HORN Y; SCHECHTER P J; MARTON L J. Phase I-II Clinical Trial with Alpha-Difluoromethylornithine - An Inhibitor of Polyamine Biosynthesis. Eur. J. Cancer Clin. Oncol. (23, No. 8, 1103-07, 1987) 7 Tab. 22 Ref.
20. In Vivo Evaluation of a Colon-Specific Drug Delivery system: An Absorption Study of Theophylline from Capsules Coated with Azo Polymers in Rats. Pharmaceutical Res. 12(2):244-247, 1995.
21. KELLOFF GJ. BOONE CW, CROWELL JA. STEELE VE. LUBET R. SIGMAN CC. Chemopreventive Drug Development: Perspectives and Progress. Bethesda, MD. CIDU. Natl. Cancer Inst. 85-98. 1994.
22. KENDRA K L. Estrogenic and antiestrogenic regulation of cell proliferation and tissue growth. Diss Ahstr Int[B]. (1988). Vol. 48, No. 7, pp. 1904.
23. KENDRA, KARI L.: KAIZENELLENBOGEN. BENITA S. An evaluation of the involvement of polyamines in modulating MCF-7 human breast cancer cell proliferation and progesterone receptor levels by estrogen and antiestrogen. J. Steroid Biochem. (1987), 28(2), 123-8.
24. LIEBERMAN HA, LACHMAN L and SCHWARTZ JB, EDS. Pharmaceutical Dosage Forms: Tablets, Vol. 3. Marcel Dekker, Inc., NY ISBN 0-8247-8300-X, 1990
25. LOVE RR, CARBONE PP. VERMA AK. GILMORE D. CAREY P. TUTSCH KD, POMPLUN M, WILDING G. Randomized Phase I Chemoprevention Dose-Seeking Study of Alpha-Difluoromethylornithine. J. Natl. Cancer Inst. 85(9):732-7, 1993.
26. LUK GD, ABELOFF MD, GRIFFIN CA, ET AL. Successful treatment with DL-alpha-difluoromethylomithine(DFMO) of established human small cell lung carcinoma implants in athymic mice. Proc. AACR 24:318, 1983.
27. MAMONT PS, DUCHESNE MC, GROVE J, ET AL. Antiproliferative properties of DL-alpha- difluoromethylomithine in cultured cells. A consequence of ornithinedecarboxylase. Biochem. Biophys. Res. Commun. 81:58-66, 1978.
28. MANNI, ANDREA; WRIGHT, CAROL. Effect of tamoxifen and .alpha.-difluoromethylomithine on clones of nitrosomethylurea-induced rat mammary tumor cells grown in soft agar culture. Cancer Res. (1983), 43(3), 1084-6.
29. MANNI, ANDREA; WRIGHT, CAROL; PONTARI, MICHEL. Polyamines and estrogen control of growth of the NMU-induced rat mammary tumor. Breast Cancer Res. Treat. (1985), 5(2), 129-36.
30. MARX M, TOWNSEND CM JR, BARRANCO SC, ET AL. Treatment of hamster pancreatic cancer with α-difluoromethylornithine, an inhibitor of polyamine biosynthesis. J. Natl. Cancer Inst. 79:543-548, 1987.
31. MCGINITY JW, ED. Aqueous Polymeric Coatings for Pharmaceutical Dosage Forms. Marcel Dekker, Inc., NY ISBN 0-8247-7907-X, 1989.
32. PRAKASH NJ, SCHECHTER PJ, MAMONT PS, ET AL. Inhibition of EMT 6 tumor growth by interference with polyamine biosynthesis; effects of alpha-difluoro-methylornithine, an irreversible inhibitor of ornithine decarboxylase. Life Sci. 26: 181-194, 1980.
33. ROBINSON JR and LEE V HL LEE, EDS. Controlled Drug Delivery: Fundamentals and Applications, 2nd ed. Marcel Dekker, Inc., NY ISBN 0-8247-7588-0, 1987.
34. RUBINSTEIN A, ET AL. In Vitro and In Vivo Analysis of Colon Specificity of Calcium Pectinate Formulations. Eur. J. Pharm. Biopharm. (1995), 41(5), pg. 291-295.
35. SCHMITT-HOFFMANN AH, HAEGELE KD. Pharmacokinetics of the Enantiomers of α-difluoromethylornithine. Annual Report of the CIFRE Convention, 1987.
36. STEELE V E; BOONE C W; KELLOFF G J. Use of agent combinations in the chemoprevention of experimental cancer. Proc.Am.Assoc.Cancer Res. (35, 85 Meet., 628, 1994).
37. TAKAMI H, UMEMOTO S, ABE O, ET AL. Effects of alpha-difluoromethylornithine (DFMO) combined with mitomycin C (MMC) in human tumors transplanted into nude mice. PROC. AACR 30:A2338, 1989.
38. TESTA B. Chiral Aspects of Drug Metabolism. TIPS, February, 1986.
39. THOMAS T; BUHARIN A; KIANG D T. Role of Polyamines in the Proliferation of Human Breast Cancer Cells. Clin.Res. (33, No. 4, 891A, 1985).
40. THOMAS, THRESIA; KIANG, DAVID T. Additive growth-inhibitory effects of DL-.alpha.-difluoromethylornithine and antiestrogens on MCF-7 breast cancer cell line. Biochem. Biophys. Res. Commun. (1987), 148(3), 1338-45.
41. THOMPSON, HENRY J.; RONAN, ANNE M. Effect of D,L-2 difluoromethylornithme and endocrine manipulation on the induction of mammary carcinogenesis by 1-methyl-1-nitrosourea. Carcinogenesis (London) (1986), 7(12), 2003-6.
42. THOMPSON, HENRY J.; RONAN, ANNE M.; RITACCO, KAREN A.; MEEKER, L. DAVID. Effect of tamoxifen and D,L-2-difluoromethylornithine on the growth, ornithine decarboxylase activity and polyamine content of mammary carcinomas induced by 1-methyl-1-nitrosourea. Carcinogenesis (London) (1986), 7(5), 837-40.
43. TOER TN. Colonic Drug Delivery. Proceed Intern. Symp.-Control Rel. Bioact. Mater, March 16 (1990), pg. 126-127, pg. 291-295.
44. VAN DEN MOOTER G, ET AL. The Relation Between Swelling Properties and Enzymatic Degradation of Azo Polymers Designed for Colon-Specific Drug Delivery. Pharmaceutical Res. 11(12): 1737-1741, 1994.
45. VAN DEN MOOTER G, ET AL. Characterization of Colon-Specific Azopolymers: A Study of the Swelling Properties and the Permeability of Isolated Polymer Fiulms. Intemat'l J. Pharmaceutics (1994), 111 pg. 127-136.
46. VANDELLI MA, ET AL. A Delayed Delivery System for the Colonic Drug Release; Proc. 1st World Mtg. APGI/APV, Budapest, 9/11, May 1995, pg. 278-279.
47. WAGNER, J. ET AL. Resolution of the enantiomers of various alpha-substituted ornithine and lysine analogs by high performance liquid chromatography with chiral eluent and by gas chromatography on chirasil-Val. Anal. Biochem. (1987), 164(1), 102-16.
48. WILDING IR, ET AL. Enteric Coated Timed Release Systems for Colonic Targeting. Intemat'l J. Pharmaceutics (1994), 111, pg. 99-102.

## Claims

1. A pharmaceutical composition for use as a medicament comprising therapeutically effective amounts of taxol, or a derivative, prodrug or metabolite thereof, and (+)-DFMO, (-)-DFMO or a mixture of (+)-DEMO and (-)-DFMO, or pharmaceutically acceptable salts thereof.

2. The pharmaceutical composition of claim 1 for use in treating, preventing, controlling the growth of and/or reducing the risk of breast cancer or tumor.

3. The pharmaceutical composition of claim 2 wherein the breast cancer or tumor is estrogen independent.

4. The pharmaceutical composition of any one of claims 1 to 3 wherein said DFMO is present in an amount of 0.01% to 90% by weight of the composition.

5. The pharmaceutical composition of any one of claims 1 to 4 further comprising a therapeutically effective amount of a cytotoxic or cytostatic agent.

6. A kit of parts comprising:
(a) a therapeutically effective amount of taxol, or a derivative, prodrug or metabolite thereof; and
(b) (+)-DFMO, (-)-DFMO, or a mixture of (+)-DFMO and (-)-DFMO, or pharmaceutically acceptable salts thereof
for use in sequential or concurrent administration as a medicament.

7. The kit of claim 6 for use in treating, preventing, controlling the growth of and/or reducing the risk of breast cancer or tumor.

8. The kit of claim 6, for use in treating a patient having or being at risk of developing breast cancer.

9. The kit of claim 6, for use in preventing breast cancer or tumor growth.

10. The kit of claim 6, for use in controlling and/or inhibiting the growth of breast cancer or tumor.

11. The kit of claim 6, for use in reducing the risk of estrogen independent breast cancer or tumor.

12. The use of
(a) a therapeutically effective amount of taxol, or a derivative, prodrug or metabolite thereof; and
(b) (+)-DFMO, (-)-DFMO, or a mixture of (+)-DFMO and (-)-DFMO, or pharmaceutically acceptable salts thereof
for the manufacture of a medicament for treating, preventing, controlling the growth of and/or reducing the risk of breast cancer or tumor.

13. The use of claim 12 wherein said DFMO is present in an amount of 0.01% to 90% by weight.

14. The use of claim 12 or 13 further comprising the use of a therapeutically effective amount of a cytotoxic or cytostatic agent.

15. The use of any one of claims 12 to 14 wherein the breast cancer or tumor is estrogen independent.

## Patentansprüche

1. Pharmazeutische Zusammensetzung zur Verwendung als ein Arzneimittel aufweisend therapeutisch wirksame Mengen an Taxol oder eines Derivats, eines Pro-Pharmakons oder eines Metaboliten davon und (+) - DFMO, (-) - DFMO oder ein Gemisch aus (+) - DFMO und (-) - DFMO oder pharmazeutisch annehmbare Salze davon.

2. Pharmazeutische Zusammensetzung nach Anspruch 1 zur Verwendung bei der Behandlung, zur Verhinderung, zur Kontrolle des Wachstums und/oder zur Verringerung des Risikos von Brustkrebs oder - Tumor.

3. Pharmazeutische Zusammensetzung nach Anspruch 2, wobei der Brustkrebs oder -Tumor östrogenunabhängig ist.

4. Pharmazeutische Zusammensetzung nach einem der Ansprüche 1 bis 3, wobei das DFMO in einer Menge von 0,01 Gewichtsprozent bis 90 Gewichtsprozent der Zusammensetzung vorliegt.

5. Pharmazeutische Zusammensetzung nach einem der Ansprüche 1 bis 4, außerdem aufweisend eine therapeutisch wirksame Menge eines zytotoxischen oder zytostatischen Mittels.

6. Kollektion von Bestandteilen aufweisend:
(a) eine therapeutisch wirksame Menge an Taxol oder eines Derivats, eines Pro-Pharmakons oder eines Metaboliten davon; und
(b) (+) - DFMO, (-) - DFMO oder ein Gemisch aus (+) - DFMO und (-) - DFMO oder pharmazeutisch annehmbare Salze davon
zur Verwendung bei einer aufeinanderfolgenden oder gleichzeitigen Verabreichung als ein Arzneimittel.

7. Kollektion nach Anspruch 6 zur Verwendung bei der Behandlung, zur Verhinderung, zur Kontrolle des Wachstums und/oder zur Verringerung des Risikos von Brustkrebs oder -Tumor.

8. Kollektion nach Anspruch 6 zur Verwendung bei der Behandlung einer Patientin, die Brustkrebs hat oder in Gefahr ist, Brustkrebs zu entwickeln.

9. Kollektion nach Anspruch 6 zur Verwendung zur Verhinderung von Brustkrebs- oder -Tumorwachstum.

10. Kollektion nach Anspruch 6 zur Verwendung zur Kontrolle und/oder zur Hemmung des Wachstums von Brustkrebs oder -Tumor.

11. Kollektion nach Anspruch 6 zur Verwendung zur Verringerung des Risikos von östrogenunabhängigem Brustkrebs oder -Tumor.

12. Verwendung
(a) einer therapeutisch wirksamen Menge an Taxol oder eines Derivats, eines Pro-Pharmakons oder eines Metaboliten davon; und
(b) von (+) - DFMO, (-) - DFMO oder eines Gemisches aus (+) - DFMO und (-) - DFMO oder von pharmazeutisch annehmbaren Salzen davon
zur Herstellung eines Arzneimittels zur Behandlung, Verhinderung, Kontrolle des Wachstums und/oder zur Verringerung des Risikos von Brustkrebs oder -Tumor.

13. Verwendung nach Anspruch 12, bei der das DFMO in einer Menge von 0,01 Gewichtsprozent bis 90 Gewichtsprozent vorliegt.

14. Verwendung nach Anspruch 12 oder 13, außerdem aufweisend die Verwendung einer therapeutisch wirksamen Menge eines zytotoxischen oder zytostatischen Mittels.

15. Verwendung nach einem der Ansprüche 12 bis 14, bei der der Brustkrebs oder -Tumor östrogenunabhängig ist.

## Revendications

1. Composition pharmaceutique destinée à être utilisée comme médicament comprenant des quantités thérapeutiquement efficaces de taxol, ou d'un dérivé, promédicament ou métabolite de celui-ci, et de (+)-DFMO, (-)-DFMO ou d'un mélange de (+)-DFMO et de (-)-DFMO , ou de sels pharmaceutiquement acceptables de celles-ci.

2. Composition pharmaceutique selon la revendication 1 destinée à être utilisée dans le traitement, la prévention, la maîtrise de la croissance de et/ou la réduction du risque de cancer du sein ou de tumeur.

3. Composition pharmaceutique selon la revendication 2, où le cancer du sein ou la tumeur est indépendant d'oestrogènes.

4. Composition pharmaceutique selon l'une quelconque des revendications 1 à 3, où ladite DFMO est présente en une quantité de 0,01 % à 90 % en masse de la composition.

5. Composition pharmaceutique selon l'une quelconque des revendications 1 à 4 comprenant en outre une quantité thérapeutiquement efficace d'un agent cytotoxique ou cytostatique.

6. Trousse de parties comprenant :
(a) une quantité thérapeutiquement efficace de taxol, ou d'un dérivé, promédicament ou métabolite de celui-ci ; et
(b) de la (+)-DFMO, (-)-DFMO ou un mélange de (+)-DFMO et de (-)-DFMO, ou des sels pharmaceutiquement acceptables de celles-ci.
destinée à être utilisée dans l'administration successive ou simultanée à titre de médicament.

7. Trousse selon la revendication 6 destinée à être utilisée dans le traitement, la prévention, la maîtrise de la croissance de et/ou la réduction du risque de cancer du sein ou de tumeur.

8. Trousse selon la revendication 6 destinée à être utilisée dans le traitement d'un patient ayant ou risquant de développer un cancer du sein.

9. Trousse selon la revendication 6 destinée à être utilisée dans la prévention du cancer du sein ou de la croissance tumorale.

10. Trousse selon la revendication 6 destinée à être utilisée dans la maîtrise et/ou l'inhibition de la croissance du cancer du sein ou d'une tumeur.

11. Trousse selon la revendication 6 destinée à être utilisée dans la réduction du risque de cancer du sein ou de tumeur indépendant d'oestrogènes.

12. Utilisation de
(a) une quantité thérapeutiquement efficace de taxol, ou d'un dérivé, promédicament ou métabolite de celui-ci ; et
(b) la (+)-DFMO, (-)-DFMO ou un mélange de (+)-DFMO et de (-)-DFMO, ou de sels pharmaceutiquement acceptables de celles-ci
pour la fabrication d'un médicament pour traiter, prévenir, maîtriser la croissance de et/ou réduire le risque de cancer du sein ou de tumeur.

13. Utilisation selon la revendication 12, où ladite DFMO est présente en une quantité de 0,01 % à 90 % en masse.

14. Utilisation selon la revendication 12 ou 13 comprenant en outre l'utilisation d'une quantité thérapeutiquement efficace d'un agent cytotoxique ou cytostatique.

15. Utilisation selon l'une quelconque des revendications 12 à 14 où le cancer du sein ou la tumeur est indépendant d'oestrogènes.
